# EUROPÄISCHE PATENTANMELDUNG

(11) **EP 4 732 815 A1**
(43) Veröffentlichungstag der Anmeldung: **29.04.2026**
(21) Anmeldenummer: 25210424.5
(22) Anmeldetag: 22.10.2025
(51) Int. Cl.: A61F 13/06, A61F 13/10

(54) **WUNDAUFLAGE FÜR FINGER ODER ZEHEN**

(30) Priorität: 28.10.2024 DE 102024131294
(71) Anmelder: Paul Hartmann AG, 89522 Heidenheim (DE)
(72) Erfinder: Streit, Iris, 89522 Heidenheim (DE)
(74) Vertreter: Paul Hartmann AG Patents & Licensing

(57) **Zusammenfassung**

Die vorliegende Erfindung betrifft eine Wundauflage für Finger oder Zehen umfassend einen Absorptionskern, eine hydrophobe Vliesschicht und eine hydrophile Vliesschicht. Die hydrophobe Vliesschicht und die hydrophile Vliesschicht sind mittels einer Ultraschallschweißnaht verbunden und bilden eine Tasche. Der Absorptionskern, der zwischen der hydrophoben Vliesschicht und der hydrophilen Vliesschicht in der Tasche eingeschlossen ist, umfasst ein absorbierendes Fasermaterial und ein superabsorbierendes Polymer. Die Grundfläche der Wundauflage besitzt eine Form, die auf zwei identischen, gleichschenkligen Trapezen basiert, die an der jeweils kürzeren Grundseite aneinandergefügt sind und wobei mindestens alle an die Grundseiten anschließenden Seiten eine konvexe Wölbung aufweisen.

## Beschreibung

Die vorliegende Erfindung betrifft eine Wundauflage für Finger oder Zehen.

Absorbierende Wundauflagen sind im Stand der Technik seit langem bekannt. Sie sollen die Wunde insbesondere vor schädlichen Umwelteinflüssen schützen und von der Wunde abgegebene Wundflüssigkeit aufnehmen. Für stark sezernierende Wunden werden heutzutage häufig Wundauflagen mit einem Anteil an superabsorbierenden Polymer (SAP) eingesetzt. Solche Wundauflagen können eine große Menge an Wundexsudat aufnehmen und halten.

Damit die absorbierende Wundauflage für diesen Zweck eingesetzt werden kann, muss sie die Wunde vollständig abdecken und es darf keine Aussparungen im Wundverband geben, die zu einem Auslaufen der Wundauflage führen könnten. Im Bereich der Finger oder Zehen ist dies besonders schwierig, insbesondere dann, wenn die Wunde den gesamten Umfang der Gliedmaße umfasst. Dann sind konventionelle, pflasterartige Wundauflagen, die mit ihrem absorbierenden Wundkissen nur einen Teil der Zehe oder des Fingers bedecken können, ungeeignet.

Die Aufgabe der vorliegenden Erfindung bestand darin eine Wundauflage zur Verfügung zu stellen, die für die Behandlung von stärker exsudierenden und großflächigeren Wunden an Fingern und insbesondere Zehen besonders gut geeignet ist.

Diese Aufgabe wird gelöst durch eine Wundauflage für Finger oder Zehen umfassend einen Absorptionskern, eine hydrophobe Vliesschicht und eine hydrophile Vliesschicht,
wobei die hydrophobe Vliesschicht und die hydrophile Vliesschicht mittels einer Ultraschallschweißnaht an ihren Rändern verbunden sind und eine Tasche bilden,
wobei der Absorptionskern ein absorbierendes Fasermaterial, insbesondere ein zellulosebasiertes Fasermaterial, und ein superabsorbierendes Polymer, insbesondere SAP-Partikel, umfasst,
und wobei der Absorptionskern zwischen der hydrophoben Vliesschicht und der hydrophilen Vliesschicht in der Tasche eingeschlossen ist.

Die erfindungsgemäße Wundauflage ist dadurch gekennzeichnet, dass die Wundauflage eine Grundfläche besitzt, deren Form auf zwei identischen, gleichschenkligen Trapezen basiert, die an der jeweils kürzeren Grundseite aneinandergefügt sind und wobei mindestens alle an die Grundseiten anschließenden Seiten eine konvexe Wölbung aufweisen. Dabei kann der Absorptionskern im Wesentlichen der Form der Wundauflage folgen und/oder die Tasche im Wesentlichen ausfüllen.

Die Form der Wundauflage hat damit im nicht angelegten Zustand eine Sanduhr-Form. Durch die konvexe Wölbung der Seiten nach außen, welche an die Grundseiten anschließen, erhält die Wundauflage eine Form, welche besonders gut an die Finger- und Zehenform anpassbar ist. Diese zusätzliche Wölbung nach außen sorgt dafür, dass die Finger- oder Zehenkuppe im angelegten Zustand der Wundauflage vollständig eingeschlossen ist. Die Wundauflage bildet eine Kappe ohne Lücken. Bei Wundauflagen, bei denen die an die Grundseiten der Trapeze anschließenden Seiten eine konkave Wölbung aufweisen, z.B. wenn eine Wundauflage auf zwei gegenüberliegenden Seiten eine ovale Aussparung hat, bildet sich beim Anlegen der Wundauflage eine kreisförmige Aussparung am gewölbten Ende der so gebildeten Kappe. Bei der vorliegenden Erfindung kann die Wundauflage auch ausgedehntere Wunden sicher abdecken und es ist sichergestellt, dass die als Wundkontaktschicht vorgesehene hydrophile Vliesschicht die Wundfläche vollständig bedeckt und der Absorptionskern über der Wundfläche angeordnet ist. Dadurch kann das Wundexsudat von der Wundauflage vollständig aufgenommen und gehalten werden. Anders als bei Wundauflagen, die als geschlossene Finger- oder Zehenkappen ausgebildet sind, ist es dabei nicht notwendig, verschiedene Größen von Wundauflagen zur Verfügung zu stellen, um der Größe des Zehs oder des Fingers Rechnung zu tragen. Die erfindungsgemäße Wundauflage kann nämlich während des Anlegens an die Form des Zehs oder des Fingers angepasst werden.

Falls nicht anders angegeben, beziehen sich die Gew.-%-Angaben auf das Gesamtgewicht der jeweiligen Schicht.

Der Absorptionskern ist in der Tasche, welche aus der hydrophoben Vliesschicht und der hydrophilen Vliesschicht gebildet wird, eingeschlossen. Der Absorptionskern umfasst ein absorbierendes Fasermaterial, wie Zellulose-Fasern, und ein superabsorbierendes Polymer (SAP), wie ein Polyacrylat. Insbesondere handelt es sich bei dem SAP vorwiegend um SAP-Partikel. In einer bevorzugten Ausführungsform bilden die Zellulose-Fasern einen Fluff, in welchen SAP-Partikel eingestreut sind. Die SAP-Partikel sind dabei bevorzugt gleichmäßig über das Volumen des Absorptionskerns verteilt.

Die Verwendung von Fluff im Absorptionskern hat den Vorteil, dass die Wundstelle besser gepolstert ist. Vor allem bei langfristigen Anwendungen, insbesondere im Zehenbereich, erhöht dies den Tragekomfort.

Der Absorptionskern kann zum Beispiel 30 Gew.-% bis 60 Gew.-% SAP-Partikel und 40 Gew.- % bis 70 Gew.- % absorbierendes Fasermaterial umfassen. Bevorzugt umfasst der Absorptionskern 40 Gew.-% bis 50 Gew.- % SAP-Partikel und 50 Gew.-% bis 60 Gew.-% absorbierendes Fasermaterial. Die Angaben beziehen sich dabei jeweils auf das Gesamtgewicht des Absorptionskerns.

Um eine gleichmäßigere Verteilung des Wundexsudats und somit eine bessere Ausnutzung der Kapazität des Absorptionskerns zu gewährleisten, kann der Absorptionskern von einer Verteilerschicht eingefasst sein. Die Verteilerschicht ist dabei sowohl zwischen Absorptionskern und der hydrophoben Vliesschicht als auch zwischen dem Absorptionskern und der hydrophilen Vliesschicht angeordnet. Die Verteilerschicht kann als geschlossene Hülle oder als offene, Umhüllung um den Absorptionskern ausgebildet sein. Die geschlossene Hülle und die offene Umhüllung können beispielsweise mittels Zuschneidens und/oder Verschweißens der um den Absorptionskern gefalteten Verteilerschicht erzeugt werden. Die Verteilerschicht besteht aus einem flüssigkeitsdurchlässigen und wasserdampfdurchlässigen Material. Dabei kann es sich um ein hydrophiles Zellulosegewebe oder ein Zellulosevlies handeln.

Der Absorptionskern kann eine Saugleistung von mindestens 50 g/100 cm², vorzugsweise von mindestens 100 g/100 cm² und insbesondere von mindestens 140 g/100 cm², gemessen nach DIN EN 13726-1:2002-06 (Kapitel 3.2), aufweisen. Diese hohen Saugleistungen können durch eine geeignete Menge an SAP-Partikeln im Absorptionskern problemlos erzielt werden. Die Saugleistung des Absorptionskerns kann maximal 220 g/100 cm², 250 g/100 cm² oder 300 g/100 cm², gemessen nach DIN EN 13726-1:2002-06 (Kapitel 3.2), betragen. Bevorzugt weist der Absorptionskern eine Saugleistung von 140 g/100 cm² bis 220 g/100 cm², gemessen nach DIN EN 13726-1:2002-06 (Kapitel 3.2), auf.

Wie bereits erwähnt kann der Absorptionskern im Wesentlichen der Form der Wundauflage folgen und damit bevorzugt ebenfalls eine Sanduhr-förmige Grundfläche besitzen. Zudem kann der Absorptionskern vorteilhafterweise die Tasche, die durch die hydrophobe Vliesschicht und die hydrophile Vliesschicht gebildet wird, im Wesentlichen ausfüllen. Jedoch dehnt sich der Absorptionskern bei Flüssigkeitsaufnahme typischerweise aus. Diese Volumenvergrößerung sollte bei der Dimensionierung des Absorptionskern im trockenen Zustand berücksichtigt werden, indem der trockene Absorptionskern die Tasche nicht komplett ausfüllt.

In einer Ausführungsform ist der äußere Rand des Absorptionskerns im trockenen Zustand höchstens 5 mm, bevorzugt höchstens 3 mm, von dem inneren Rand der Tasche, die den Absorptionskern einschließt, entfernt. Dies gilt für den gesamten Umfang des Absorptionskerns. Umschließt eine Verteilerschicht den Absorptionskern, so ist auch die Verteilerschicht höchstens 5 mm, bevorzugt höchstens 3 mm, von dem inneren Rand der Tasche, die den Absorptionskern einschließt, entfernt.

In einer weiteren Ausführungsform sind mindestens 60 %, bevorzugt 60 - 99 %, bevorzugter 70 - 98 %, insbesondere 80 - 95 % oder 80 - 90 % der Grundfläche der Tasche von dem Absorptionskern im trockenen Zustand bedeckt.

In einer weiteren Ausführungsform füllt das Volumen des Absorptionskerns und die vorzugsweise vorhandene Verteilerschicht das Volumen der Tasche, in die sie eingebracht sind, vollständig aus, wenn der Absorptionskern 100 % seiner Füllkapazität erreicht hat.

Die hydrophile Vliesschicht ist als Wundkontaktschicht vorgesehen und schließt zusammen mit der hydrophoben Vliesschicht den Absorptionskern ein. Bei Verwendung der Wundauflage ist die hydrophile Vliesschicht daher wundseitig angeordnet. Die hydrophile Vliesschicht ist flüssigkeitsdurchlässig, so dass das Wundexsudat von der Wunde zu dem Absorptionskern gelangen kann.

Die hydrophile Vliesschicht umfasst bevorzugt eine Mischung aus Kunststofffasern und Zellulosefasern. Bei Kunststofffasern handelt es sich um ein vollsynthetisches Material.

Die hydrophile Vliesschicht kann beispielsweise aus 60 bis 70 Gew.-% Kunststofffasern und 30 bis 40 Gew.-% Fasern auf Zellulosebasis bestehen. Insbesondere kann die hydrophile Vliesschicht aus etwa 64 Gew.-% Kunststofffasern und etwa 36 Gew.-% Fasern auf Zellulosebasis bestehen.

In einer besonders bevorzugten Ausführungsform ist die hydrophile Vliesschicht zweilagig aufgebaut und besteht aus einer inneren Vliesstofflage und einer äußeren Vliesstofflage. Die innere Vliesstofflage bildet einen Teil der Innenseite der Tasche und besteht aus Kunststofffasern und Fasern auf Zellulosebasis. Die äußere Vliesstofflage bildet einen Teil der Außenseite der Tasche und besteht aus einem Vlies aus Kunststofffasern. Die äußere Vliesstofflage der hydrophilen Vliesschicht kann dabei wundseitig angeordnet sein und atraumatische Eigenschaften aufweisen, d. h. die hydrophile Vliesschicht haftet nicht an der Wunde und kann auf sanfte und schmerzfreie Weise aus der Wunde entfernt werden. Die innere Vliesstofflage und die äußere Vliesstofflage können durch Wärmeeinwirkung miteinander verbunden werden.

Die innere Vliesstofflage kann aus 35 bis 45 Gew.-% Kunststofffasern und 55 bis 65 Gew.-% Fasern auf Zellulosebasis bestehen. Insbesondere kann die innere Vliesstofflage aus etwa 40 Gew.-% Kunststofffasern und etwa 60 Gew.-% Fasern auf Zellulosebasis bestehen. Die innere und die äußere Vliesstofflage können zusammen aus 60 bis 70 Gew.-% Kunststofffasern und 30 bis 40 Gew.-% Fasern auf Zellulosebasis bestehen, wie bereits in Bezug auf die (gesamte) hydrophile Vliesstofflage erwähnt. Die hydrophile Vliesschicht kann ein Flächengewicht von 25 bis 50 g/m², vorzugsweise 40 bis 50 g/m² und insbesondere von etwa 45 g/m² aufweisen.

Die hydrophobe Vliesschicht ist im Wesentlichen flüssigkeitsundurchlässig. Damit verhindert sie, dass die von dem Absorptionskern aufgenommene Flüssigkeit aus der Wundauflage entweichen kann.

Die hydrophobe Vliesschicht umfasst bevorzugt Kunststofffasern. In einer besonders bevorzugten Ausführungsform kann die hydrophobe Vliesschicht ausschließlich aus Kunststofffasern bestehen. Bevorzugt weist die hydrophobe Vliesschicht ein Flächengewicht von 20 bis 50 g/m², vorzugsweise von 20 bis 30 g/m² und insbesondere von etwa 25 g/m² auf. Ein geringes Flächengewicht kann die Atmungsaktivität und die Anpassungsfähigkeit der Wundauflage erhöhen.

Normalerweise haben die hydrophile Vliesschicht und die hydrophobe Vliesschicht im Wesentlichen die gleiche geometrische Form und Abmessung. Eine solche Ausbildung ist herstellungstechnisch leicht umsetzbar. Denkbar ist jedoch auch, dass die wundseitig angeordnete hydrophile Vliesschicht eine geringere Abmessung hat, so dass im angelegten Zustand der Wundauflage die Ränder der hydrophilen und hydrophoben Vliesschicht die Tasche bilden können, ohne dass die hydrophobe Vliesschicht unter Zug steht. Damit übt die Wundauflage keinen Druck auf die Gliedmaße aus, an die sie angelegt ist und der Tragekomfort ist erhöht.

In einer besonders bevorzugten Ausführungsform umfassen die hydrophile Vliesschicht und die hydrophobe Vliesschicht Kunststofffasern aus demselben Material. Die Kunststofffasern weisen typischerweise thermoplastische Eigenschaften auf. Da die Kunststofffasern in beiden Lagen dann dieselbe Schmelztemperatur besitzen, kann der Schweißprozess in einem engeren Energiebereich stattfinden, als wenn verschiedene Kunststofffasern verwendet würden. Dadurch kann die Schweißnaht schmaler ausgebildet sein, während die Schweißnaht nichts von ihrer Festigkeit einbüßt. Somit kann die Schweißnaht eine geringere Fläche einnehmen und die Flexibilität der Wundauflage gegenüber einer Wundauflage mit breiteren Schweißnähten ist verbessert, insbesondere ist die Wundauflage dadurch leichter zu biegen. Diese Eigenschaft der erfindungsgemäßen Wundauflage ist besonders vorteilhaft im Bereich der Finger- und Zehenkuppen, wo die Wundauflage im angelegten Zustand die stärkste Wölbung besitzt. Ein anderer Vorteil ist, dass die Wundauflage durch die dünneren Schweißnähte geschmeidiger ist und damit einen erhöhten Tragekomfort aufweist.

Um dieses vorteilhafte Schweißen zu ermöglichen, können die hydrophile Vliesschicht und die hydrophobe Vliesschicht vorteilhaft jeweils mindestens 30 Gew.-%, vorzugsweise mindestens 40 Gew.-%, noch bevorzugter mindestens 50 Gew.-% und insbesondere mindestens 60 Gew.- % Kunststofffasern enthalten. In einer Ausführungsform enthalten die hydrophile und die hydrophobe Vliesschicht unterschiedliche Mengen der Kunststofffasern. Beispielsweise kann die hydrophobe Vliesschicht mindestens 90 Gew.-% der Kunststofffasern enthalten, während die hydrophile Vliesschicht zwischen 50 und 70 Gew.-% der Kunststofffasern enthalten kann. Vorzugsweise weist die hydrophile Vliesschicht 60 bis 70 Gew-% Polypropylenfasern, und 30 bis 40 Gew-% Viskosefasern auf.

In einer bevorzugten Ausführungsform bestehen die in der hydrophilen Vliesschicht und der hydrophoben Vliesschicht enthaltenen Kunststofffasern aus Acrylnitril-Butadien-Styrol (ABS), Polyamid (PA), Polylactid (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) oder Polyvinylchlorid (PVC). Vorzugsweise bestehen die in der hydrophilen Vliesschicht und der hydrophoben Vliesschicht enthaltenen Kunststofffasern aus Polyamid (PA), Polyethylenterephthalat (PET), Polyethylen (PE), Polypropylen (PP) oder Polyvinylchlorid (PVC). Insbesondere bestehen die in der hydrophilen Vliesschicht und der hydrophoben Vliesschicht enthaltenen Kunststofffasern aus Polypropylen.

Ebenso haben die Fasern, insbesondere die Kunststofffasern, der hydrophilen und hydrophoben Vliesschicht bevorzugt denselben Durchmesser und/oder dieselbe Länge.
Abbildung 1 zeigt einen Querschnitt einer erfindungsgemäßen Wundauflage
Abbildung 2 zeigt eine Draufsicht einer erfindungsgemäßen Wundauflage
Abbildung 3 zeigt eine Ausführungsform der erfindungsgemäßen Wundauflage im angelegten Zustand

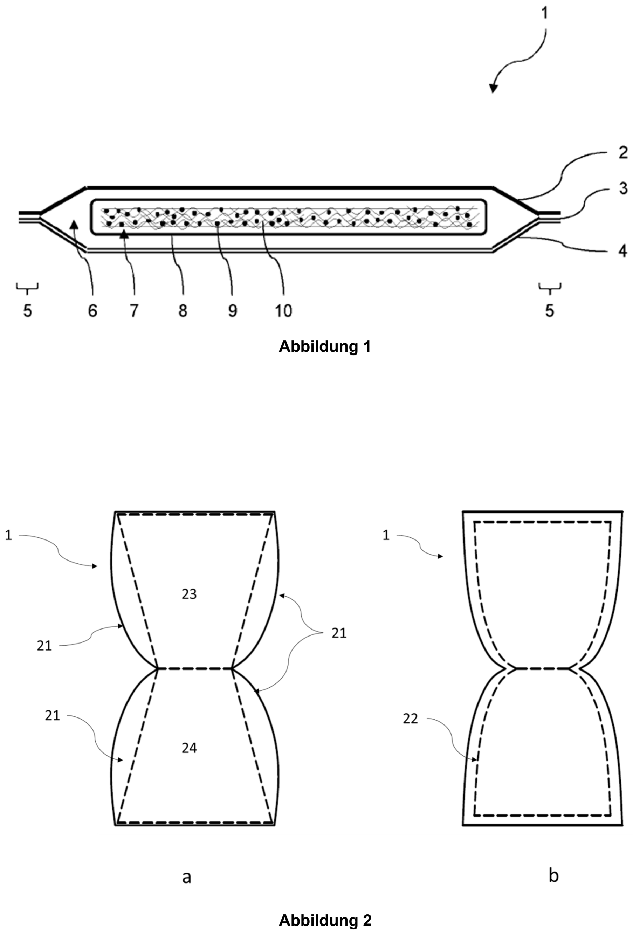

Abbildung 1 zeigt eine Ausführungsform der erfindungsgemäßen Wundauflage 1 im Querschnitt. Die Wundauflage 1 umfasst eine hydrophobe Vliesschicht 2, die aus Polypropylenfasern besteht. Die hydrophobe Vliesschicht 2 ist im angelegten Zustand wundabseitig angeordnet und bildet die äußerste Schicht der Wundauflage.

Die Wundauflage 1 umfasst des Weiteren eine hydrophile Vliesschicht, die gemäß der bevorzugten Ausführungsform von Abbildung 1 aus zwei Vliesstofflagen 3, 4 besteht, die durch Wärmeeinwirkung miteinander verbunden sind. Die innere Vliesstofflage 3 besteht aus einer Mischung von Polypropylenfasern und Viskosefasern, vorzugsweise in einem Verhältnis von etwa 40 Gew.-% Polypropylenfasern und etwa 60 Gew.-% Viskosefasern. Die äußere Vliesstofflage 4 besteht dagegen ähnlich wie die hydrophobe Vliesschicht 2 ausschließlich aus Polypropylenfasern. Vorzugsweise bestehen die innere und äußere Vliesstofflage 3, 4 zusammen damit durchschnittlich aus etwa 64 Gew.-% Polypropylenfasern und etwa 36 Gew.- % Viskosefasern. Die hydrophile Vliesschicht 3, 4 ist der Wunde zugewandt, wenn die Wundauflage 1 während der Wundbehandlung über der Wunde positioniert wird. Die hydrophile Vliesschicht 3, 4 bildet die für den direkten Wundkontakt vorgesehene Unterseite der Wundauflage 1 und stellt somit eine Wundkontaktschicht der Wundauflage 1 dar.

Eine Ultraschallschweißnaht 5 verbindet einen Rand der hydrophoben Vliesschicht 2 und einen Rand der hydrophilen Vliesschicht 3, 4 miteinander, so dass die hydrophobe Vliesschicht 2 und die hydrophile Vliesschicht 3, 4 eine Tasche 6 bilden. In der Tasche 6 befindet sich ein Absorptionskern 7, der von einer Verteilerschicht aus hydrophilem Vlies 8 aus Zellulosefasern umschlossen ist. Der Absorptionskern 7 besteht aus einem Fluff aus Zellulosefasern 10 und superabsorbierenden Polymerpartikeln 9 auf Polyacrylatbasis. Vorzugsweise besteht der Absorptionskern aus 40 bis 50 Gew.-% superabsorbierenden Polymerpartikeln und 50 bis 60 Gew.-% Zellulosefasern. Der Absorptionskern ist im trockenen Zustand sehr weich und die SAP-Partikel sind nur lose in den Flockenzellstoff eingebettet.

Die durch die hydrophile und hydrophobe Vliesschicht 2, 3, 4 gebildete Umhüllung hält den Absorptionskern zusammen.

Bei der Wundbehandlung tritt das Wundexsudat aufgrund der saugfähigen und flüssigkeitsverteilenden Eigenschaften der hydrophilen Vliesschicht 3, 4 sowie der Verteilerschicht 8 in die Wundauflage 1 ein. Schließlich wird das Wundexsudat in dem Absorptionskern 7 gebunden. Die hydrophobe Vliesschicht 2 verhindert das Austreten von Wundexsudat aus der Wundauflage 1, wenn der Absorptionskern 7 mit Wundexsudat gesättigt ist. Die doppellagige Struktur der hydrophilen Vliesschicht 3, 4 verleiht der Wundauflage 1 atraumatische Eigenschaften, ohne die Saugfähigkeit und Flüssigkeitsverteilung zu beeinträchtigen. Die Wundauflage 1 enthält an ihren Außenflächen keinen Klebstoff. Zur Fixierung der Wundauflage 1 an der Wundstelle kann ein medizinisches Klebeband oder eine Bandage verwendet werden.

In Abbildung 2a und 2b ist eine Draufsicht der erfindungsgemäßen Wundauflage 1 dargestellt. Die Grundfläche der Wundauflage 1 basiert auf zwei identischen, gleichschenkligen Trapezen 23, 24, die an der jeweils kürzeren Grundseite aneinandergefügt sind. Die an die Grundseiten anschließenden Seiten weisen eine konvexe Wölbung 21 auf. Durch diese nach außen gerichteten Ränder 21 erhält die Wundauflage 1 eine Sanduhr-Form. Der Absorptionskern 7 folgt ebenfalls der Sanduhr-Form und füllt die Tasche, die durch die hydrophobe Vliesschicht und die hydrophile Vliesschicht gebildet wird, weitestgehend aus. Die Kontur des Absorptionskerns 7 ist als gestrichelte Linie 22 eingezeichnet.

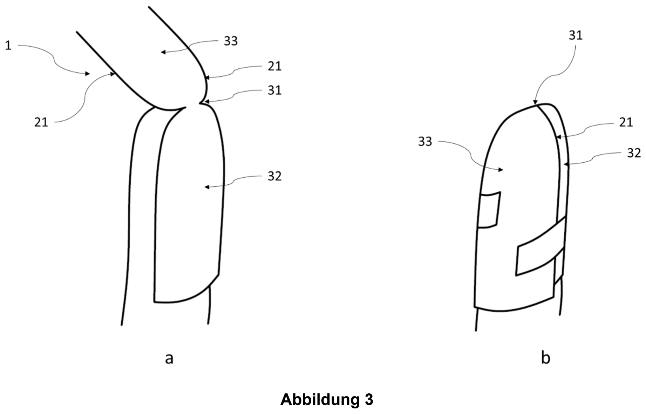

Abbildung 3a und 3b zeigt die Wundauflage 1 im angelegten Zustand.

Die Wundauflage 1 wird so über dem Finger oder der Zehe ausgerichtet, dass die Verjüngung 31 in der Mitte der Wundauflage über der Finger- oder Zehenkuppe angeordnet ist. Die beiden symmetrischen Seiten 32, 33 der Wundauflage 1 werden nacheinander nach unten geklappt, siehe Abbildung 3a, und so um den Finger oder die Zehe gelegt, dass die beiden Seiten eine Kappe bilden, die die Kuppe und die Wunde an dem Finger oder der Zehe komplett einschließen, siehe Abbildung 3b. Beim Anlegen wird der Umfang des Fingers oder der Zehe berücksichtigt. Die abgerundeten Ränder 21 der Wundauflage werden entsprechend eng um den Finger oder die Zehe gezogen. Die Wundauflage 1 umschließt nun den Finger oder die Zehe komplett und kann mittels eines Befestigungsmittels fixiert werden. Wegen der Sanduhr-Form nach Anspruch 1 bildet auch der Absorptionskern im angelegten Zustand eine Kappe um die Finger- oder Zehenkuppe. Damit kann das Wundexsudat vollständig aufgefangen und in der Wundauflage 1 gehalten werden.

## Patentansprüche

1. Wundauflage (1) für Finger oder Zehen,
umfassend einen Absorptionskern, eine hydrophobe Vliesschicht (2) und eine hydrophile Vliesschicht (3,4),
wobei die hydrophobe Vliesschicht und die hydrophile Vliesschicht (3,4) mittels einer Ultraschallschweißnaht (5) verbunden sind und eine Tasche (6) bilden,
wobei der Absorptionskern (7) ein absorbierendes Fasermaterial (10) und ein superabsorbierendes Polymer (9) umfasst,
und wobei der Absorptionskern (7) zwischen der hydrophoben Vliesschicht (2) und der hydrophilen Vliesschicht (3,4) in der Tasche (6) eingeschlossen ist,
**dadurch gekennzeichnet, dass** die Wundauflage (1) eine Grundfläche besitzt, deren Form auf zwei identischen, gleichschenkligen Trapezen (23, 24) basiert, die an der jeweils kürzeren Grundseite aneinandergefügt sind und wobei mindestens alle an die Grundseiten anschließenden Seiten eine konvexe Wölbung (21) aufweisen.

2. Wundauflage (1) nach Anspruch 1, wobei die hydrophobe Vliesschicht (2) und die hydrophile Vliesschicht (3,4) Kunststofffasern aus demselben Material umfassen.

3. Wundauflage (1) nach Anspruch 2, wobei die Kunststofffasern aus Polypropylen bestehen.

4. Wundauflage (1) nach mindestens einem der vorgenannten Ansprüche, wobei der Absorptionskern (7) im Wesentlichen der Form der Wundauflage (1) folgt und/oder die Tasche (6) im Wesentlichen ausfüllt.

5. Wundauflage (1) nach mindestens einem der vorgenannten Ansprüche, wobei die Wundauflage (1) eine Verteilerschicht (8) aus einem flüssigkeitsdurchlässigen und wasserdampfdurchlässigen Material umfasst, und der Absorptionskern (7) so von der Verteilerschicht (8) eingefasst ist, dass die Verteilerschicht (8) sowohl zwischen dem Absorptionskern (7) und der hydrophoben Vliesschicht (2) als auch zwischen dem Absorptionskern (7) und der hydrophilen Vliesschicht (3,4) angeordnet ist.

6. Wundauflage (1) nach mindestens einem der vorgenannten Ansprüche, wobei der äußere Rand des Absorptionskerns (7) höchstens 5 mm von dem inneren Rand der Tasche (6), die den Absorptionskern (7) einschließt, entfernt ist.

7. Wundauflage (1) nach mindestens einem der vorgenannten Ansprüche, wobei mindestens 60 % der Grundfläche der Tasche (6), die den Absorptionskern (7) einschließt, von dem Absorptionskern (7) im trockenen Zustand bedeckt ist.

8. Wundauflage (1) nach mindestens einem der vorgenannten Ansprüche, wobei die hydrophile Vliesschicht (3,4) aus einer inneren Vliesstofflage (3) und einer äußeren Vliesstofflage (4) besteht,
und wobei die innere Vliesstofflage (3) aus Kunststofffasern und Fasern auf Zellulosebasis besteht und wobei die äußere Vliesstofflage (4) aus Kunststofffasern besteht.

9. Wundauflage (1) nach mindestens einem der vorgenannten Ansprüche, wobei die hydrophile Vliesschicht (3,4) aus 60 bis 70 Gew.-% Kunststofffasern und 30 bis 40 Gew.-% Fasern auf Zellulosebasis, insbesondere aus etwa 64 Gew.-% Kunststofffasern und etwa 36 Gew.-% Fasern auf Zellulosebasis, besteht.

10. Wundauflage (1) nach mindestens einem der vorgenannten Ansprüche, wobei die hydrophile Vliesschicht (3,4) Polypropylenfasern und Viskosefasern umfasst.

11. Wundauflage (1) nach mindestens einem der vorgenannten Ansprüche, wobei der Absorptionskern (7) eine Saugleistung von 140g/100cm² bis 220g/100cm² aufweist.

12. Wundauflage (1) nach mindestens einem der vorgenannten Ansprüche, wobei der Absorptionskern (7) 40 Gew.-% bis 50 Gew.- % SAP-Partikel (9) und 50 Gew.-% bis 60 Gew.-% absorbierendes Fasermaterial (10) umfasst.
